# EUROPEAN PATENT SPECIFICATION

(11) **EP 4 236 869 B1**
(45) Date of publication and mention of the grant of the patent: **15.01.2025**
(21) Application number: 21754847.8
(22) Date of filing: 06.08.2021
(51) Int. Cl.: A61F 2/24, A61B 17/00

(54) **ARTICULATED PROSTHESIS FOR A TRICUSPID OR MITRAL VALVE**
GELENKPROTHESE FÜR EINE TRIKUSPIDAL- ODER MITRALKLAPPE
PROTHÈSE ARTICULÉE POUR VALVE TRICUSPIDE OU MITRALE

(30) Priority: 30.10.2020 IT 202000025879
(43) Date of publication of application: 06.09.2023
(73) Proprietor: Star Tric S.r.l., 20121 Milano (IT)
(72) Inventor: DE BONIS, Michele, 20063 Cernusco Sul Naviglio MI (IT); ALFIERI, Ottavio, 25121 Brescia BS (IT); LAPENNA, Elisabetta, 20063 Cernusco Sul Naviglio MI (IT); ZANOTTI, Daniele, 32040 Comelico Superiore BL (IT); GARD, Marco, 10031 Borgomasino TO (IT); PASSANANTE, Eugenio Maria, 20147 Milano MI (IT)
(74) Representative: Barbaro, Gaetano
(86) International application number: PCT/IB2021/057257
(87) International publication number: WO 2022/090818

(56) References cited:
- US-A1- 2019 008 643
- US-A1- 2019 350 702
- US-A1- 2020 163 757

## Description

### TECHNICAL FIELD

This disclosure relates to the repair of heart valves exhibiting valve regurgitation. More particularly, the invention relates to an apparatus suitable for a less invasive repair of a heart valve using an articulated prosthesis deliverable through a catheter, for leaflets of a tricuspid or mitral heart valve.

### BACKGROUND

The most common type of tricuspid valve disease is functional tricuspid regurgitation (TR), which is mainly due to the dilatation of the tricuspid annulus secondary to right ventricular enlargement. Later in the course of the disease, tethering of the tricuspid leaflets may also occur because of the displacement of the papillary muscles inside the right ventricle. When functional TR is due to both severe annular dilatation and leaflet tethering, annuloplasty alone is unlikely to be successful. Similarly, TR caused by prolapse or flail of multiple leaflets, as typically seen in post-traumatic and severe degenerative TR, cannot be rectified by a simple annuloplasty procedure.

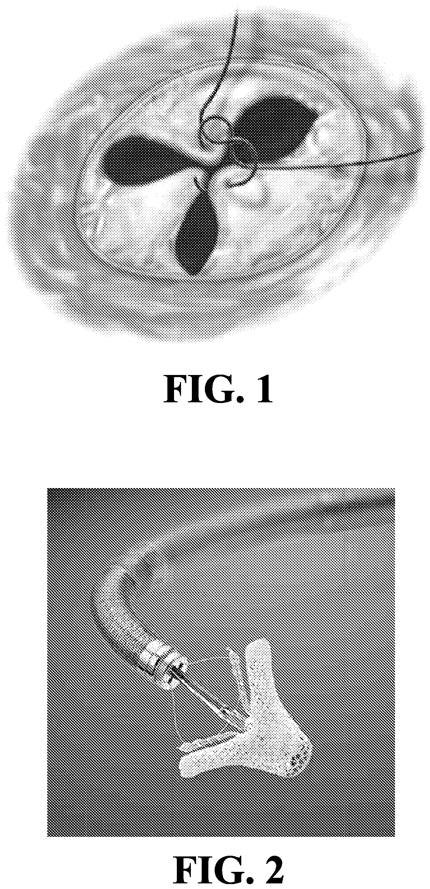

In order to achieve an effective and durable repair, the so-called 'clover technique' has been proposed. This technique consists in suturing together the central part of the free edges of the tricuspid leaflets, producing a 'clover'-shaped valve. A pictorial representation of a tricuspid valve treated according to this technique is depicted in figure 1. Clinical results obtained with this technique are reported in:
- *"*The 'clover technique' as a novel approach for correction of post-traumatic tricuspid regurgitation", O. Alfieri, M. De Bonis et al., Journal of Thoracic and Cardiovascular Surgery, 2003; Vol. 126, No. 1, pages 75-79;
- *"*A novel technique for correction of severe tricuspid valve regurgitation due to complex lesions. " De Bonis M, Lapenna E et al. Eur. J. Cardiothorac. Surg. 2004 May;25(5):760-5.
- *"*Four-leaflet clover repair of severe tricuspid valve regurgitation due to complex lesions", E. Lapenna, M. De Bonis et al., Journal of Cardiovascular Medicine, 2008, Vo. 9 No. 8, pages 847-849;
- *"*The clover technique for the treatment of complex tricuspid valve insufficiency: midterm clinical and echocardiographic results in 66 patients", E. Lapenna, M. De Bonis et al., European Journal of Cardio-thoracic Surgery, 37 (2010), 1297-1303;
- *"*Long-term results (up to 14 years) of the clover technique for the treatment of complex tricuspid valve regurgitation", De Bonis M, Lapenna E, et al. Eur. J. Cardiothorac. Surg. 2017 Feb. 23. doi: 10.1093/ejcts/ezx027.

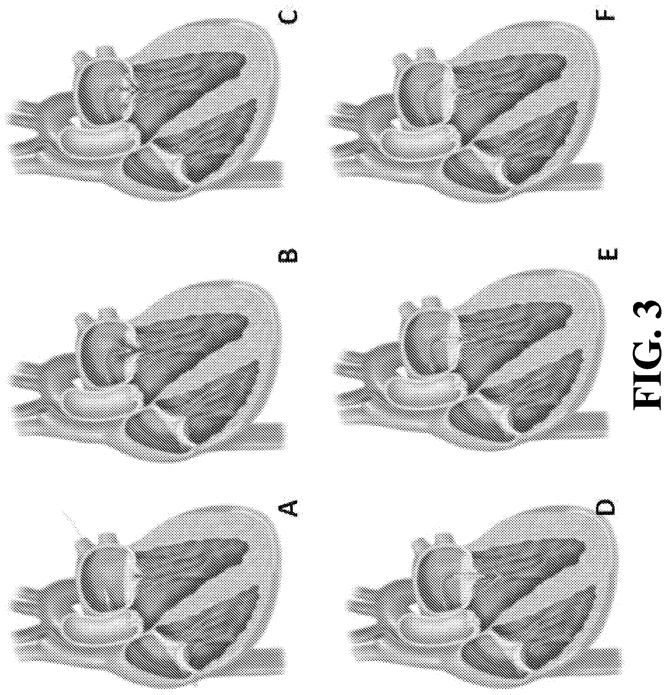

Devices for catching valve leaflets of a mitral valve as well as of a tricuspid valve, are marketed with the commercial namse MITRACLIP^{™} and TRICLIP^{™}. This prior device, that can be introduced into the heart through a catheter by a vascular approach or throughout a small incision in the chest, comprises a fastener applicator of the type shown in figure 2. The sequence of operations to be performed for implanting a MITRACLIP^{™} fastener is shown in figure 3. In the depicted case the heart valve is the mitral valve, though the same observations hold *mutatis mutandis* also for the tricuspid valve. Using a catheter, the MITRACLIP^{™} fastener is inserted in a folded configuration into the heart; when the catheter is close to the heart valve, the fastener is deployed like an umbrella to catch the leaflets of the valve, and it is subsequently closed to grip the leaflets together. Finally, the MITRACLIP^{™} fastener is left closed in the heart for keeping the leaflets together, thus reducing valve regurgitation.

Unfortunately, tests carried out by the applicant have shown that this prior applicator with two arms is unable to catch simultaneously all the 3 leaflets of the tricuspid valve and, therefore, its efficacy in treating tricuspid regurgitation is very limited. In presence of very dilated tricuspid annulus, catching also of just 2 leaflets of the tricuspid valve is rather difficult with the MitraClip^{™} system. When more MitraClips^{™} are implanted in the aim of improving the competence of the tricuspid valve, the risk of tricuspid stenosis significantly increases by reducing too much the orifice of the repaired tricuspid valve. Without being bound to a theory, the increased risk of stenosis may be due to the fact that the central portions of the leaflets of the heart valve are folded at a right angle in respect to the plane of the heart valve in order to be gripped together one against the other. The leaflets assume an unnatural configuration overstretched toward the center of the valve plane, thus the light therebetween is reduced and the risk of stenosis is increased.

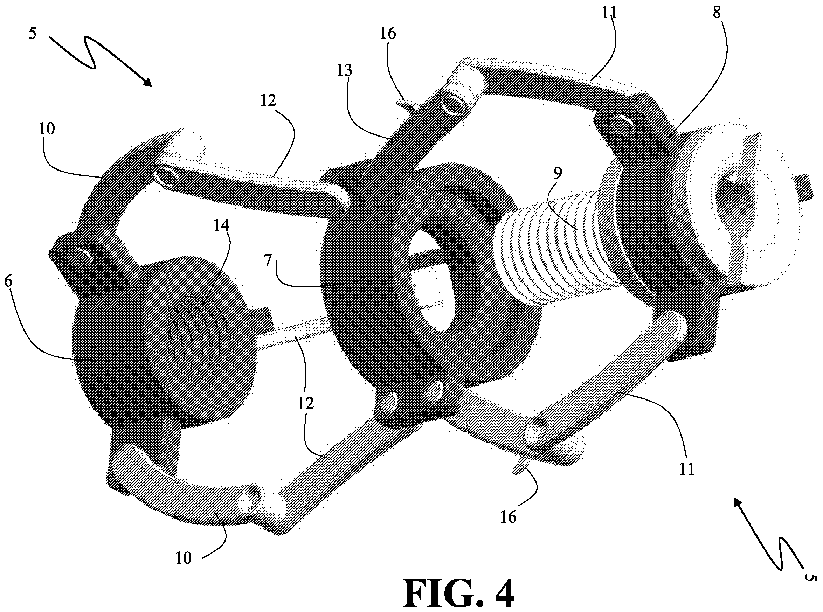

A relevant improvement in respect to the MITRACLIP fastener is represented by the articulated prosthesis of figures 4 to 6B, which is conceived so as to grip simultaneously all the three leaflets of a tricuspid valve so as to make them lay distended fully in the valve plane and to assume a final configuration as in the common surgical procedure.

The articulated prosthesis 5 in a distended configuration is illustrated in figure 4. It comprises:
- a distal annular cylindrical portion 6,
- a central annular cylindrical portion 7,
- a proximal annular cylindrical portion having a cylindrical collar 8 and a screw 9 engaged into the collar 8 and supported thereby so as to be free to rotate along a longitudinal axis,
- a first plurality of arms 10 hinged to an outer side surface of the distal annular cylindrical portion 6,
- a second plurality of arms 11 hinged to an outer side surface of the collar 8,
- a third plurality of arms 12 each of which is hinged to an outer side surface of the central annular cylindrical portion 7, from one end, and to a corresponding end of a arm 10 from the opposite end,
- a fourth plurality of arms 13 each of which is hinged to an outer side surface of the central annular cylindrical portion 7, from one end, and to a corresponding end of a arm 11 from the opposite end.

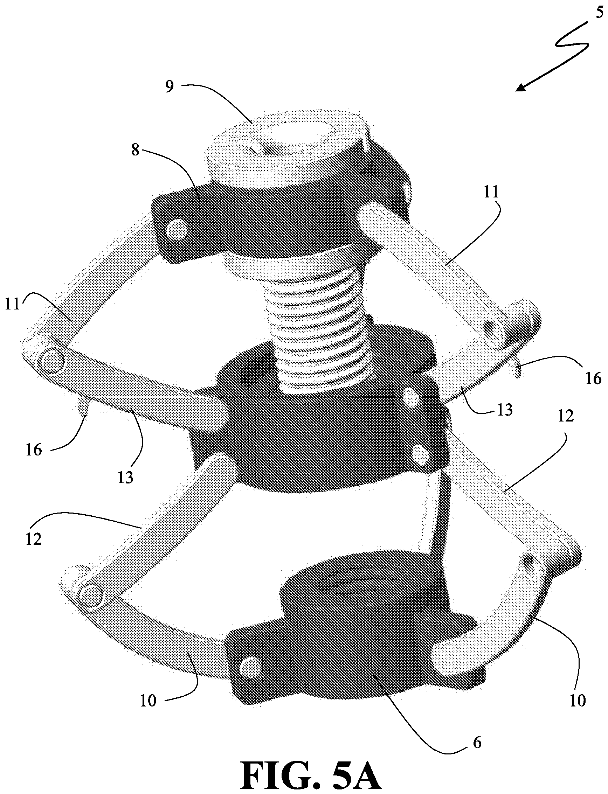

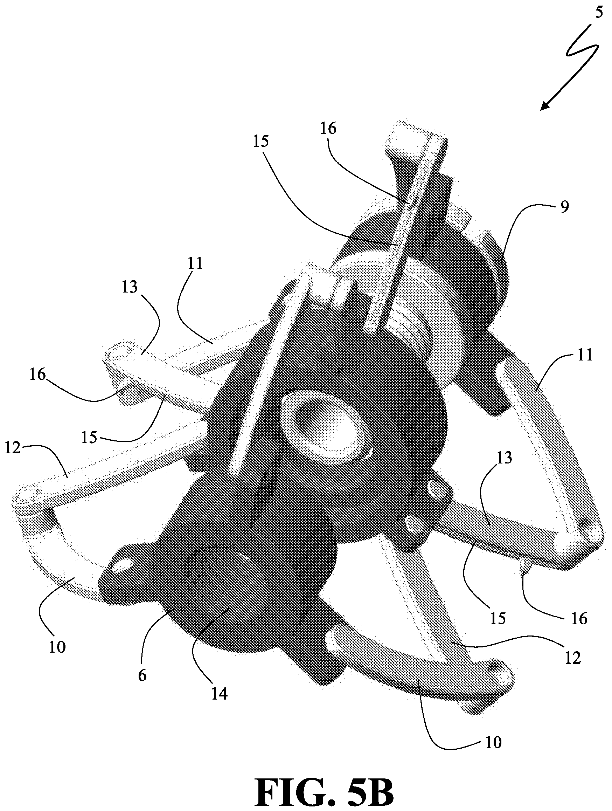

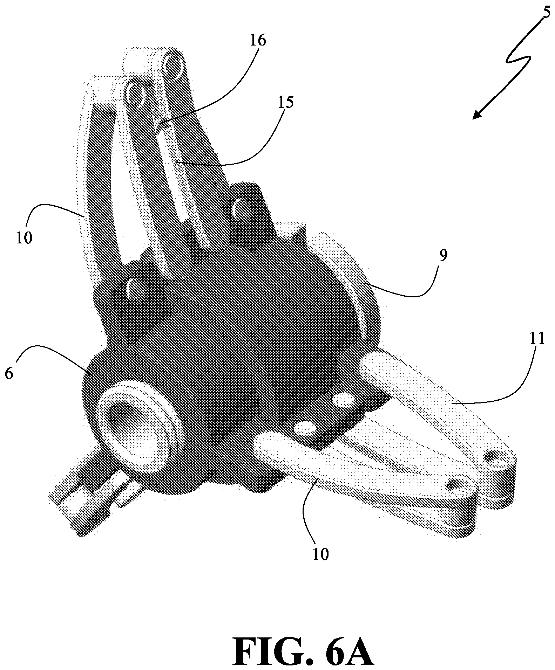

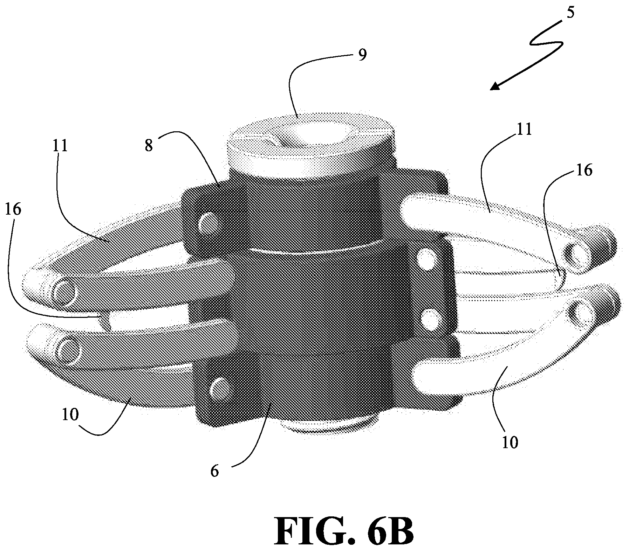

The distal portion 6 has an internal screw thread 14 configured to engage with the screw 9 when the articulated prosthesis 5 passes from the distended configuration of figure 5 to the expanded configuration of figures 6A and 6B. The central portion 7 has a through hole to let the screw 9 pass therethrough. By screwing the screw 9 in the distal portion 6, the arms 10, 11, 12, 13 are extended and the arms 12 and 13 grab between them the leaflets of the heart valve. When the screw 9 is tightened in the screw thread 14, the articulated prosthesis 5 holds firmly the heart valve leaflets and is left in place as a prosthesis, that may be easily removed by a surgeon if necessary.

The arms shall be configured to catch firmly the leaflets and preventing them from slipping out.

In order to provide a good gripping of the leaflets, at least one of the facing arms 12 and 13 has a knurling 15 and a pointed projection 16 configured to spike the tissue of the corresponding leaflet. In the depicted embodiment only the arms 13 are provided with the knurling 15 and the projection 16, though they may be realized also on some or all of the arms 12 for catching even better the leaflets of the heart valve.

The relative position of the pointed projection 16 on the arm 13 may be established to spike a heart valve leaflet as close as possible to the tip, as shown in figure 1 and as it would be done in the classic surgical technique. This result may be attained for example by placing the pointed projections 16 at about the middle of the arm 13.

Document US2020/163757 discloses an articulated prosthesis, left in the patient's heart for repairing a tricuspid or mitral valve, conceived so as to grip simultaneously all the three leaflets of the tricuspid valve, or the two of the mitral valve, so as to make them lay distended fully in the valve plane and assume a final configuration as in the common surgical procedure.

### SUMMARY

Even if this articulated prosthesis provides better results in respect to other similar prosthesis, the way in which the leaflets are caught may still be improved. More in detail, the applicants found that it is possible to realize an articulated prosthesis configured so as to pull the leaflets of a human valve towards the center of the valve, as it would be done with the surgical procedure illustrated in figure 1.

This outstanding result is attained with an articulated prosthesis as defined in the enclosed claim 1.

Further embodiments are defined in the enclosed claims.

### BRIEF DESCRIPTION OF THE DRAWINGS

Figure 1 schematically depicts a typical configuration of a tricuspid valve after the so-called "clover technique" surgical intervention.
Figure 2 depicts a prior fastener for leaflets of a mitral or tricuspid valve.
Figure 3 shows various steps for implanting the so-called MITRACLIP^{™} fastener to leaflets of a heart valve.
Figure 4 depicts an articulated prosthesis in a distended configuration.
Figures 5A and 5B depict the articulated prosthesis of figure 4 in a half-extended configuration.
Figures 6A and 6B depict the articulated prosthesis of figure 4 in an extended configuration with the screw of the proximal portion tightly screwed into the distal portion.
Figures 7A to 7C depict the articulated prosthesis of this disclosure in an extended configuration with the screw of the proximal portion tightly screwed into the distal portion.

### DETAILED DESCRIPTION OF EXEMPLARY EMBODIMENTS

In the ensuing description reference will be made to the repair of a tricuspid valve, though the same observations hold *mutatis mutandis* for the repair of a mitral valve. For this reason, the articulated prosthesis shown in the figures has three rows of arms regularly disposed around circular side surfaces of the catching device in order to grip the three leaflets of a tricuspid valve. Nevertheless, the depicted prosthesis 5 could be made with only two rows of arms for gripping the two leaflets of a mitral valve, or even with four or more rows of arms for better catching the leaflets of any heart valve.

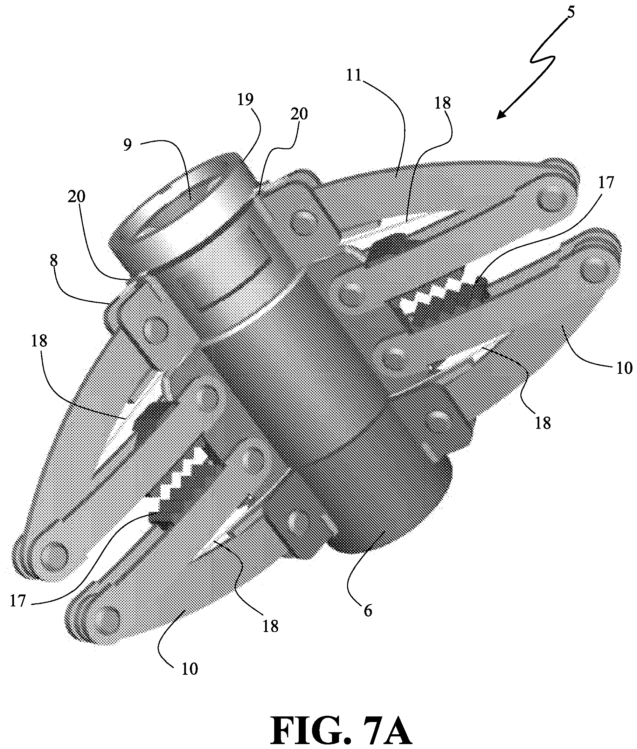

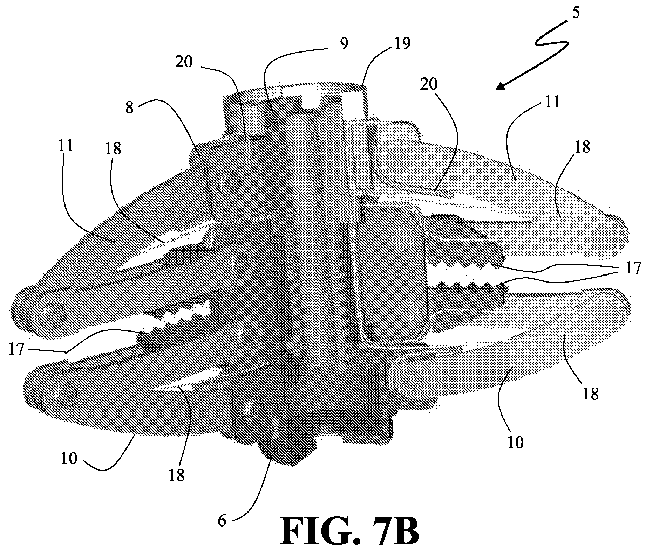

The articulated prosthesis of this disclosure is depicted in figures 7A to 7C, wherein the same components bear the same reference numbers. As the prior prosthesis of figures 4 to 6B, the articulated prosthesis of this disclosure has a distal annular portion, a central annular portion and a proximal annular portion; a first plurality of arms hinged to an outer side surface of the distal annular portion; a second plurality of arms hinged to an outer side surface of the proximal annular portion; a third plurality of arms each of which is hinged to an outer side surface of the central annular portion, from one end, and to a corresponding end of a arm of the first plurality, from an opposite end; and a fourth plurality of arms each of which is hinged to an outer side surface of the central annular portion, from one end, and to a corresponding end of a arm of the second plurality, from an opposite end.

As in the prior prosthesis, the distal annular portion has an internal screw thread, the central annular portion defines a through hole for letting the proximal annular portion pass through, and the proximal annular portion has:
- a cylindrical collar on a side surface of which the second plurality of arms is hinged, and
- a screw engaged into the collar and supported thereby so as to be free to rotate along a longitudinal axis of the screw, the screw being configured to engage with the internal screw thread.

The articulated prosthesis is movable from a distended configuration (not shown in the figures), in which all the arms hinged in correspondence of their ends are distended, to an expanded configuration, in which the proximal annular portion is threaded throughout the central annular portion and is engaged with the distal annular portion, the arms of the third plurality and fourth plurality being configured to hold therebetween leaflets of a tricuspid or mitral valve.

Differently from the prior prosthesis, the arms of the third plurality and/or of the fourth plurality are equipped with sliders 17. Each slider 17 is configured to be suspended between a respective pair of arms to slide back and forth in a radial direction of the articulated prosthesis when the prosthesis is in the expanded configuration. The sliders 17 have a catching surface configured to grasp by friction leaflets of a tricuspid or mitral valve, and have at least one respective wire 18 fixed thereto and configured to pull the slider 17 radially outwards, when the wire 18 is pulled in a first direction, and to pull the slider 17 radially inwards, when the wire 18 is pulled in the opposite direction.

According to one aspect, each slider 17 has a surface with teeth configured to grasp a leaflet of a tricuspid or mitral valve by cooperating with a facing slider 17.

According to one aspect, not shown in the drawings, there may be sliders 17 in the form of a plate having a surface with a knurling configured to grasp by friction a leaflet of a tricuspid or mitral valve.

According to one aspect, shown in the figures, the wires 18 enter in the proximal annular portion 8, cross axially the central annular portion 7, run radially outwards and are fixed to a slider 17, turn around an outer pin that hinges an end of one arm of the second plurality of arms to at least one arm of the fourth plurality of arms, run radially towards the center, cross axially the central annular portion 7 and then exit from the proximal annular portion 8. Once the leaflets of a valve are caught between the teeth of facing sliders 17, the wires 18 are pulled so as the leaflets are dragged towards the center of the valve as in the common surgical procedure. When the leaflets are distended, the wires 18 are blocked using a holding mechanism 20 so as the leaflets are kept distended and pulled as they were sewn together (as show in figure 1).

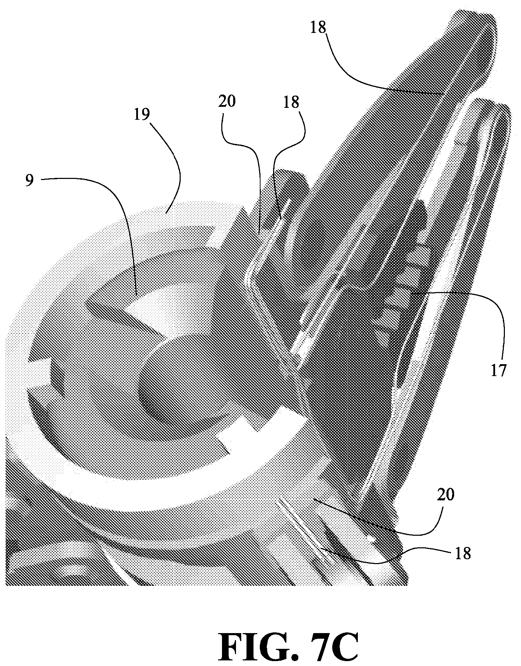

According to one aspect, illustrated in the detail view of figure 7C, the holding mechanism comprises an element 20 fixed to the cylindrical collar 8. The element has a slit and is positioned so as to intercept a path along which at least one wire 18 is disposed. When the leaflets are caught between two facing sliders 17, the wires 18 are pulled so as to drag the leaflets towards the center of the valve, the wires 18 are forcibly inserted in the slit of the element 20 and are kept pulled. Finally, the wires 18 are cut by a surgeon so as its terminations are held firmly in the slit of the element 20.

According to one aspect, the holding mechanism is equipped with a ring 20 mounted around the head of the screw 9 and configured so as, when the screw 9 is tightened, the ring 20 is held against the cylindrical collar 8 and cooperates to it to sandwich the terminations of the wires 18.

According to one aspect, the number of arms of the second plurality and third plurality is twice the number of arms of the first plurality and fourth plurality, wherein each arm of the fourth plurality is hinged together with two arms of the second plurality, and each arm of the third plurality is hinged together with two arms of the first plurality.

## Claims

1. An articulated prosthesis (5) for a tricuspid or a mitral valve, comprising:
a distal annular portion (6), a central annular portion (7) and a proximal annular portion (8);
a first plurality of arms (10) hinged to an outer side surface of said distal annular portion (6);
a second plurality of arms (11) hinged to an outer side surface of said proximal annular portion (8);
a third plurality of arms (12) each of which is hinged to an outer side surface of said central annular portion (7), from one end, and to a corresponding end of a arm of said first plurality (10), from an opposite end;
a fourth plurality (13) of arms each of which is hinged to an outer side surface of said central annular portion (7), from one end, and to a corresponding end of a arm of said second plurality (11), from an opposite end;
said distal annular portion (6) having an internal screw thread (14), said proximal annular portion (8) having:
- a cylindrical collar (8) on a side surface of which said second plurality of arms (11) is hinged, and
- a screw (9) engaged into said collar (8) and supported thereby so as to be free to rotate along a longitudinal axis of the screw (9), the screw (9) being configured to engage with said internal screw thread (14),
said central annular portion (7) defining a through hole for letting said proximal annular portion (8) pass through;
the articulated prosthesis (5) being movable from a distended configuration, in which all said arms hinged in correspondence of their ends are distended, to an expanded configuration, in which said proximal annular portion (8) is threaded throughout said central annular portion (7) and is engaged with said distal annular portion (6), the arms of said third plurality (12) and fourth plurality (13) being configured to hold therebetween leaflets of a tricuspid or mitral valve,
**characterized in that**
said arms of said third plurality (12) and/or of said fourth plurality (13) are equipped with sliders (17), each slider of said sliders (17) being configured to be suspended between a respective pair of said arms of said third plurality (12) and/or of said fourth plurality (13) and to slide back and forth along said respective pair of arms in a radial direction of said articulated prosthesis (5) when the prosthesis (5) is in said expanded configuration;
each slider of said sliders (17) has a catching surface configured to grasp by friction leaflets of a tricuspid or mitral valve, and has at least one respective wire (18) fixed thereto and configured to pull said slider (17) radially outwards, when the wire (18) is pulled in a first direction, and to pull said slider (17) radially inwards, when the wire (18) is pulled in a second direction opposite to said first direction;
said proximal annular portion (8) being equipped with a holding mechanism configured to hold pulled said at least one wire (18).

2. The articulated prosthesis (5) of claim 1, wherein said holding mechanism comprises:
an element with a slit fixed to said cylindrical collar (8), said element with the slit being placed to intercept a path along which said at least one wire (18) is disposed, wherein said at least one wire (18) is held pulled when both its terminations are forcibly inserted in said slit.

3. The articulated prosthesis (5) of claim 1 or 2, wherein said at least one wire (18) enters in said proximal annular portion (8), crosses axially said central annular portion (7) in said first direction, runs radially outwards and it is fixed to said slider (17), turns around an outer pin that hinges an end of at least one arm of said second plurality of arms (11) to at least one arm of said fourth plurality of arms (13), runs radially inwards, crosses axially said central annular portion (7) in said second direction and then exits from said proximal annular portion (8).

4. The articulated prosthesis (5) of one of claims from 1 to 3, wherein each slider of said sliders (17) has a surface with teeth configured to grasp a leaflet of a tricuspid or mitral valve.

5. The articulated prosthesis (5) of one of claims from 1 to 3, wherein said sliders (17) are in the form of a plate having a surface with a knurling (15) configured to grasp by friction a leaflet of a tricuspid or mitral valve.

6. The articulated prosthesis (5) of one of claims 1 to 5, wherein
the number of said arms of said second plurality (11) and third plurality (12) is twice the number of arms of said first plurality (10) and fourth plurality (13),
each arm of said fourth plurality (13) is hinged together with two arms of said second plurality (11) at said opposite end,
each arm of said third plurality (12) is hinged together with two arms of said first plurality (10) at said opposite end.

7. The articulated prosthesis (5) of one of claims 1 to 6 for a tricuspid valve, wherein each of said pluralities of arms comprises three arms or an integer multiple of three arms.

## Patentansprüche

1. Gelenkprothese (5) für eine Trikuspidal- oder eine Mitralklappe, umfassend:
einen distalen ringförmigen Abschnitt (6), einen mittleren ringförmigen Abschnitt (7) und einen proximalen ringförmigen Abschnitt (8);
eine erste Vielzahl von Armen (10), die an einer äußeren Seitenoberfläche des distalen ringförmigen Abschnitts (6) gelenkig angebracht sind;
eine zweite Vielzahl von Armen (11), die an einer äußeren Seitenoberfläche des proximalen ringförmigen Abschnitts (8) gelenkig angebracht sind;
eine dritte Vielzahl von Armen (12), von denen jeder an einem Ende an einer äußeren Seitenoberfläche des mittleren ringförmigen Abschnitts (7) und an einem gegenüberliegenden Ende an einem entsprechenden Ende eines Arms der ersten Vielzahl (10) gelenkig angebracht ist;
eine vierte Vielzahl (13) von Armen, von denen jeder an einem Ende an einer äußeren Seitenoberfläche des mittleren ringförmigen Abschnitts (7) und an einem gegenüberliegenden Ende an einem entsprechenden Ende eines Arms der zweiten Vielzahl (11) gelenkig angebracht ist;
wobei der distale ringförmige Abschnitt (6) ein inneres Schraubengewinde (14) aufweist, wobei der proximale ringförmige Abschnitt (8) aufweist:
- einen zylindrischen Kragen (8), an dessen Seitenoberfläche die zweite Vielzahl von Armen (11) gelenkig angebracht ist, und
- eine Schraube (9), die in den Kragen (8) eingreift und dadurch gestützt wird, um frei zu sein, um entlang einer Längsachse der Schraube (9) zu rotieren, wobei die Schraube (9) konfiguriert ist, um in das innere Schraubengewinde (14) einzugreifen,
wobei der mittlere ringförmige Abschnitt (7) ein Durchgangsloch definiert, zum Hindurchlassen des proximalen ringförmigen Abschnitts (8);
wobei die Gelenkprothese (5) aus einer ausgezogenen Konfiguration, in der alle entsprechend ihren Enden gelenkig angebrachten Arme ausgezogen sind, in eine erweiterte Konfiguration bewegbar ist, in der der proximale ringförmige Abschnitt (8) durch den mittleren ringförmigen Abschnitt (7) durchweg geschraubt ist und mit dem distalen ringförmigen Abschnitt (6) in Eingriff steht, wobei die Arme der dritten Vielzahl (12) und der vierten Vielzahl (13) konfiguriert sind, um dort zwischen Klappensegeln einer Trikuspidal- oder Mitralklappe gehalten zu werden,
**dadurch gekennzeichnet, dass**
die Arme der dritten Vielzahl (12) und/oder der vierten Vielzahl (13) sind mit Gleitern (17) ausgestattet, wobei jeder Gleiter der Gleiter (17) konfiguriert ist, um zwischen einem jeweiligen Paar der Arme der dritten Vielzahl (12) und/oder der vierten Vielzahl (13) aufgehängt zu werden und entlang des jeweiligen Paars von Armen in radialer Richtung der Gelenkprothese (5) vor und zurück zu gleiten, wenn die Prothese (5) in der erweiterten Konfiguration ist;
jeder Schieber der Schieber (17) eine Fangoberfläche aufweist, die konfiguriert ist, um durch Reibung die Klappensegel einer Trikuspidal- oder Mitralklappe zu greifen, und mindestens einen entsprechenden Draht (18) aufweist, der daran befestigt ist und konfiguriert ist, um den Schieber (17) radial nach außen zu ziehen, wenn der Draht (18) in einer ersten Richtung gezogen wird, und den Schieber (17) radial nach innen zu ziehen, wenn der Draht (18) in einer zweiten Richtung gezogen wird, die der ersten Richtung entgegengesetzt ist;
wobei der proximale ringförmige Abschnitt (8) mit einem Haltemechanismus ausgestattet ist, der konfiguriert ist, um den mindestens einen Draht (18) gezogen zu halten.

2. Gelenkprothese (5) nach Anspruch 1, wobei der Haltemechanismus umfasst:
ein Element mit einem Schlitz, das an dem zylindrischen Kragen (8) befestigt ist, wobei das Element mit dem Schlitz angeordnet ist, um einen Weg abzuscheiden, entlang dessen der mindestens eine Draht (18) eingerichtet ist, wobei der mindestens eine Draht (18) gezogen gehalten wird, wenn seine beiden Enden in den Schlitz zwangsweise eingeführt sind.

3. Gelenkprothese (5) nach Anspruch 1 oder 2, wobei der mindestens eine Draht (18) in den proximalen ringförmigen Abschnitt (8) eintritt, den mittleren ringförmigen Abschnitt (7) in der ersten Richtung axial kreuzt, radial nach außen verläuft und an dem Schieber (17) befestigt ist, sich um einen äußeren Stift dreht, der ein Ende von mindestens einem Arm der zweiten Vielzahl von Armen (11) mit mindestens einem Arm der vierten Vielzahl von Armen (13) gelenkig verbindet, radial nach innen verläuft, den mittleren ringförmigen Abschnitt (7) in der zweiten Richtung axial kreuzt und dann aus dem proximalen ringförmigen Abschnitt (8) austritt.

4. Gelenkprothese (5) nach einem der Ansprüche 1 bis 3, wobei jeder Schieber der Schieber (17) eine Oberfläche mit Zähnen aufweist, die konfiguriert ist, um ein Klappensegel einer Trikuspidal- oder Mitralklappe zu greifen.

5. Gelenkprothese (5) nach einem der Ansprüche 1 bis 3, wobei die Schieber (17) in Form einer Platte sind, die eine Oberfläche mit einer Rändelung (15) aufweist, die konfiguriert ist, um durch Reibung ein Klappensegel einer Trikuspidal- oder Mitralklappe zu greifen.

6. Gelenkprothese (5) nach einem der Ansprüche 1 bis 5, wobei
die Anzahl der Arme der zweiten Vielzahl (11) und der dritten Vielzahl (12) doppelt so groß wie die Anzahl der Arme der ersten Vielzahl (10) und der vierten Vielzahl (13) ist,
jeder Arm der vierten Vielzahl (13) an dem gegenüberliegenden Ende mit zwei Armen der zweiten Vielzahl (11) gelenkig verbunden ist,
Jeder Arm der dritten Vielzahl (12) an dem gegenüberliegenden Ende mit zwei Armen der ersten Vielzahl (10) gelenkig verbunden ist.

7. Gelenkprothese (5) nach einem der Ansprüche 1 bis 6 für eine Trikuspidalklappe, wobei jede der Vielzahl von Armen drei Arme oder ein ganzzahliges Vielfaches von drei Armen umfasst.

## Revendications

1. Prothèse à charnière (5) pour une valve tricuspide ou mitrale, comprenant :
une partie annulaire distale (6), une partie annulaire centrale (7) et une partie annulaire proximale (8) ;
une première pluralité de bras (10) articulés à une surface latérale extérieure de ladite partie annulaire distale (6) ;
une deuxième pluralité de bras (11) articulés à une surface latérale extérieure de ladite partie annulaire proximale (8) ;
une troisième pluralité de bras (12) dont chacun est articulé à une surface latérale extérieure de ladite partie annulaire centrale (7), à partir d'une extrémité, et à une extrémité correspondante d'un bras de ladite première pluralité (10), à partir d'une extrémité opposée ;
une quatrième pluralité (13) de bras dont chacun est articulé à une surface latérale extérieure de ladite partie annulaire centrale (7), à partir d'une extrémité, et à une extrémité correspondante d'un bras de ladite deuxième pluralité (11), à partir d'une extrémité opposée ;
ladite partie annulaire distale (6) ayant un filetage interne (14), ladite partie annulaire proximale (8) ayant :
- un collier cylindrique (8) sur une surface latérale duquel ladite deuxième pluralité de bras (11) est articulée, et
- une vis (9) en prise dans ledit collier (8) et supportée par celui-ci de manière à pouvoir tourner librement le long d'un axe longitudinal de la vis (9), la vis (9) étant conçue pour entrer en prise dans ledit filetage interne (14),
ladite partie annulaire centrale (7) définit un trou de passage pour laisser passer ladite partie annulaire proximale (8) ;
la prothèse à charnière (5) étant mobile depuis une configuration distendue, dans laquelle tous les bras articulés en correspondance de leurs extrémités sont distendus, à une configuration dilatée, dans laquelle la partie annulaire proximale (8) est enfilée à travers ladite partie annulaire centrale (7) et est en prise dans la partie annulaire distale (6), les bras de la troisième pluralité (12) et de la quatrième pluralité (13) étant conçus pour maintenir entre eux les valvules d'une valve tricuspide ou mitrale,
**caractérisé en ce que**
lesdits bras de ladite troisième pluralité (12) et/ou de ladite quatrième pluralité (13) sont équipés de curseurs (17), chaque curseur desdits curseurs (17) étant conçu pour être suspendu entre une paire respective desdits bras de ladite troisième pluralité (12) et/ou de ladite quatrième pluralité (13) et pour glisser d'avant en arrière le long de ladite paire respective de bras dans une direction radiale de ladite prothèse à charnière (5) lorsque la prothèse (5) est dans ladite configuration dilatée ;
chaque curseur desdits curseurs (17) a une surface d'accrochage conçue pour saisir par friction les valvules d'une valve tricuspide ou mitrale, et a au moins un fil respectif (18) fixé à celui-ci et conçu pour tirer ledit curseur (17) radialement vers l'extérieur, lorsque le fil (18) est tiré dans une première direction, et pour tirer ledit curseur (17) radialement vers l'intérieur, lorsque le fil( 18) est tiré dans une seconde direction opposée à ladite première direction ;
ladite partie annulaire proximale (8) est équipée d'un mécanisme de maintien conçu pour retenir ledit au moins un fil (18).

2. Prothèse à charnière (5) selon la revendication 1, dans laquelle ledit mécanisme de maintien comprend :
un élément avec une fente fixé au collier cylindrique (8), ledit élément avec la fente étant placé pour intercepter un chemin le long duquel ledit au moins un fil (18) est disposé, dans laquelle ledit au moins un fil (18) est maintenu tiré lorsque ses deux terminaisons sont insérées de force dans ladite fente.

3. Prothèse à charnière (5) selon la revendication 1 ou 2, dans laquelle ledit au moins un fil (18) entre dans ladite partie annulaire proximale (8), traverse de manière axiale ladite partie annulaire centrale (7) dans ladite première direction, s'étend radialement vers l'extérieur et est fixé audit curseur (17), tourne autour d'un axe extérieur qui articule une extrémité d'au moins un bras de ladite deuxième pluralité de bras (11) à au moins un bras de ladite quatrième pluralité de bras (13), s'étend radialement vers l'intérieur, traverse de manière axiale ladite partie annulaire centrale (7) dans ladite seconde direction et sort ensuite de ladite partie annulaire proximale (8).

4. Prothèse à charnière (5) selon l'une des revendications 1 à 3, dans laquelle chaque curseur desdits curseurs (17) a une surface avec des dents conçues pour saisir une valvule d'une valve tricuspide ou mitrale.

5. Prothèse à charnière (5) selon l'une des revendications de 1 à 3, dans laquelle lesdits curseurs (17) sont sous la forme d'une plaque dont la surface présente un moletage (15) conçu pour saisir par friction une valvule d'une valve tricuspide ou mitrale.

6. Prothèse à charnière (5) selon l'une des revendications 1 à 5, dans laquelle
le nombre desdits bras de la deuxième pluralité (11) et de la troisième pluralité (12) est le double du nombre de bras de la première pluralité (10) et de la quatrième pluralité (13),
chaque bras de ladite quatrième pluralité (13) est articulé ensemble avec deux bras de ladite deuxième pluralité (11) à ladite extrémité opposée,
chaque bras de ladite troisième pluralité (12) est articulé ensemble avec deux bras de ladite première pluralité (10) à ladite extrémité opposée.

7. Prothèse à charnière (5) selon l'une des revendications 1 à 6 pour une valve tricuspide, dans laquelle chacune desdites pluralités de bras comprend trois bras ou un multiple entier de trois bras.
